# EUROPÄISCHE PATENTSCHRIFT

(11) **EP 4 518 934 B1**
(45) Veröffentlichungstag und Bekanntmachung des Hinweises auf die Patenterteilung: **06.05.2026**
(21) Anmeldenummer: 23722301.1
(22) Anmeldetag: 25.04.2023
(51) Int. Cl.: A61M 16/04, A61B 1/005, A61B 1/018

(54) **MEDIZINISCHES INSTRUMENT FÜR DIE INTUBATION**
MEDICAL INSTRUMENT FOR INTUBATION
INSTRUMENT MÉDICAL POUR INTUBATION

(30) Priorität: 04.05.2022 EP 22171524
(43) Veröffentlichungstag der Anmeldung: 12.03.2025
(73) Patentinhaber: Albert-Ludwigs-Universität Freiburg, 79098 Freiburg (DE)
(72) Erfinder: SCHICK, Martin, 79098 Freiburg (DE); PETZOLD, Ralf, 79098 Freiburg (DE)
(74) Vertreter: Rösler, Uwe
(86) Internationale Anmeldenummer: PCT/EP2023/060745
(87) Internationale Veröffentlichungsnummer: WO 2023/213612

(56) Entgegenhaltungen:
- EP-A1- 3 554 604
- WO-A1-2017/079434
- WO-A1-2020/247386
- US-A1- 2009 050 146
- US-A1- 2013 317 300
- US-B2- 10 758 708
- US-B2- 9 888 832

## Beschreibung

### Technisches Gebiet

Die Erfindung bezieht sich auf ein medizinisches Instrument zum Intubieren eines Patienten mit einem einen Hohlkanal einschließenden Endotrachealtubus, kurz ETT, sowie einem längs des Hohlkanals angeordneten, wenigstens in einem axialen Teilbereich plastisch verformbaren Führungsstab, mit einem distalen und proximalen Stabende, an dessen distalen Stabende ein bidirektional verformbares Führungselement angebracht ist, mit einem distalen und einem proximalen Elementende, dessen proximales Elementende am distalen Stabende gefügt ist, und das mit einem Steuermittel in Wirkverbindung steht.

Für die Sicherung des Atemweges für eine Allgemeinanästhesie oder in der Notfall- bzw. Intensivmedizin ist und bleibt die Intubationsnarkose, kurz ITN, ein Standardprocedere, für das verschiedene Hilfsmittel benötigt werden, um einen Beatmungsschlauch bzw. Endotrachealtubus, kurz ETT, in die Luftröhre des Patienten zu platzieren. Hierbei muss der ETT durch den offenen Mund, Rachen und nachfolgend zwischen den Stimmbändern hindurch in die Luftröhre (Trachea) vorgeschoben werden. Bei diesem Procedere ist die Sicht auf die Stimmbänder, welche in aller Regel unter Zuhilfenahme eines Laryngoskops hergestellt wird, elementar. Während dieser Phase kann der Patient nicht beatmet werden, so dass ein zügiges und reibungsloses Procedere für den Patienten sehr wichtig, wenn nicht gar überlebenswichtig ist. Erst über den korrekt platzierten ETT kann der Patient sicher beatmet werden.

Ist die Intubation auf Anhieb nicht oder nur erschwert möglich, so spricht man von einer schwierigen Atemwegssicherung bzw. schwierigen Intubation. Bei Erwachsenen tritt dieser Fall in 3-10% aller durchgeführten Intubationsfällen auf, siehe Ruetzler K, Smereka J, Abelairas-Gomez C, Frass M, Dabrowski M, Bialka S, et al., Comparison of the new flexible tip bougie catheter and standard bougie stylet for tracheal intubation by anesthesiologists in different difficult airway scenarios: a randomized crossover trial. BMC Anesthesiol. 2020;20(1):90.

Allein in Deutschland werden pro Jahr ca. 24 Mio. Operationen durchgeführt, von denen etwa 70% durch eine Allgemeinanästhesie unterstützt und begleitet werden, so dass ca. 0,5 bis 1,6 Millionen Patienten pro Jahr in Deutschland von dieser Problematik betroffen sind.

Zur Erleichterung und Unterstützung der Einführung und Platzierung des ETT im Rachen sowie durch die Stimmbänder hindurch in den oberen Luftröhrenbereich hineinragend, wird ein sogenannter Führungsstab (engl. bougie, stylet) entweder zur Formgebung innerhalb des ETT platziert oder anstelle des ETT in die Luftröhre vorab eingeführt, um unmittelbar nachfolgend den ETT über den bereits eingelegten Führungsstab in die Luftröhre vorzuschieben. Während des gesamten Intubationsprozesses kann der Patient nicht beatmet werden. Zur Sicherstellung der Sauerstoffversorgung des Patienten kann es während des Intubationsprozesses immer wieder nötig werden diesen abzubrechen und den Patienten z.B. mit einer Maske zu beatmen.

Der Einsatz eines Führungsstabes ist vor allem auch in jenen Fällen besonders vorteilhaft, in denen zwar, zumeist unter Verwendung eines Laryngoskops, eine gute Sicht auf die Stimmbänder möglich ist, jedoch trotz einer geeigneten Patientenkopflage die orale, pharyngeale sowie die tracheale Atemwegsachse des Patienten ungünstig zueinander orientiert sind, so dass der weiche und produktionsbedingt vorgeformte Tubus in nicht geeigneter Weise platzierbar ist.

Der Führungsstab wird in diesen Fällen an die anatomischen Gegebenheiten individuell empirisch vorgebogen, was häufig dazu führt, dass mehrere Intubationsversuche mit im ETT einliegenden, vorgebogenen Führungsstab vorgenommen werden müssen, dessen Biegung nach jedem misslungenen Intubationsversuch angepasst werden muss, bis der richtige Winkel gefunden worden ist und der ETT in die Trachea platziert werden kann. Dieses Procedere kann mitunter mehrere Minuten benötigen.

Eine weitere Anwendung des Führungsstabes kommt bei der sogenannten Rapid Sequence Induction (RSI) zum Tragen. Dieser Prozess beinhaltet eine schnelle Intubation von Patienten nach der Narkoseeinleitung, welche z.B. hochgradig von einer Aspiration gefährdet sind, d.h. Erbrechen von Mageninhalt und anschließendem Eindringen dieser Bestandteile in die Lunge. Um hier eine schnelle und erfolgreiche Intubation zu ermöglichen, wird bei dem ersten Versuch ein Führungsstab standardmäßig in den ETT platziert und anhand der mutmaßlichen Anatomie vorgeformt. Auch hier kann es, wie vorstehend erwähnt, vorkommen, dass der Intubationsvorgang abgebrochen werden und der Führungsstab samt ETT neu vorgebogen werden muss. Hierbei besteht nicht nur die Gefahr einer Sauerstoffminderversorgung durch die fehlende Beatmung, sondern zusätzlich einer potenziell tödlichen Aspiration von Mageninhalt.

Nach einer erfolgreichen Platzierung des ETT inkl. Führungsstab, üblicherweise kurz vor oder nach der Stimmbandebene, wird der Führungsstab durch eine zweite Person entweder schrittweise zurückgezogen oder vollständig entfernt. Bei diesem Vorgang kann es vorkommen, dass durch diese Manipulation der korrekt platzierte ETT aufgrund der zwischen dem ETT und dem Führungsstab vorherrschenden hohen Reibungskräften verrutscht und dadurch nicht mehr in die Trachea einführbar ist. Den Führungsstab in situ wieder in den ETT einzuführen gelingt in den wenigsten Fällen, so dass der Vorgang abgebrochen und der Prozess von Anfang an neu begonnen werden muss, mit all den damit vorstehend erläuterten Risiken.

Der Führungsstab wird für die Mindestausstattung eines Anästhesiearbeitsplatzes der Deutschen Gesellschaft für Anästhesiologie und Intensivmedizin gefordert (S1 Leitlinie Atemwegsmanagement, siehe www.awmf.org).

### Stand der Technik

Neben der plastischen Verformbarkeit des Führungsstabes, durch die eine Vorbiegung des Führungsstabes manuell vorgenommen werden kann, um dessen Einführung und Platzierung durch den Rachen sowie die Stimmbänder hindurch in den oberen Luftröhrenbereich zu erleichtern, weisen sehr wenige moderne Führungsstäbe darüber hinaus jeweils einen kontrolliert verformbaren, distalen Stabendbereich auf, dessen räumliche Krümmung zum Zwecke einer erleichterten und Patienten schonenden Einführung des Führungsstabes veränderbar ist.

In der Druckschrift EP 3 065 803 B1 ist ein Führungsstab bzw. Bougie offenbart, der einen rohrförmigen, plastisch verformbaren Hauptschaft aufweist, an dessen distalen Ende eine bewegliche Spitze angebracht ist, die mittels eines innerhalb des Hauptschaftes längsbeweglich gelagerten Steuergliedes in einer Verformungsebene bidirektional verformbar ist. Hierzu sieht der rohrförmige Hauptschaft eine sich in Längsrichtung erstreckende Ausnehmung vor, durch die mit dem Steuerglied verbundene Vorsprünge ragen, die für den Bediener manuell zugänglich sind.

Die Druckschrift EP 3 528 878 B1 beschreibt einen gelenkigen Mandrin zur Anwendung mit einem endotrachealen Tubus, wobei der Mandrin über einen flexiblen, distalen Spitzenabschnitt verfügt, der in einer ersten Richtung vorgespannt ist und über einen Steuerdraht, der den distalen Spitzenabschnitt mit einem proximalen Mandrinabschnitt verbindet, durch entsprechende Zugwirkung in eine zur ersten Richtung entgegengesetzten zweiten Richtung verformbar ist.

Ein zum vorstehenden Mandrin sehr ähnlicher Führungsstab ist der Druckschrift WO 2007/138569 A2 zu entnehmen, der einen starren sowie einen sich daran anschließenden flexiblen Stababschnitt aufweist, an dessen distalen Ende ein, um eine Achse schwenkbar gelagertes Schwenkelement angebracht ist, zu dessen einseitiger Auslenkung ein Zugkräfte übertragendes Drahtseil vorgesehen ist, das einerseits mit dem Schwenkelement und andererseits mit einem proximalseitig am Führungsstab angebrachten Hebelmechanismus verbunden ist. Zur Intubation wird der Führungsstab gemeinsam mit dem diesem umgebenden Endotracheal-Tubus oral in den Patienten eingeführt.

Die Druckschrift WO 2017/079434 A1 offenbart einen Führungsstab mit einer elastisch verformbaren distalen Führungsstabspitze, an der wenigstens ein, vorzugsweise mehrere Zugkräfte übertragende Drähte angebracht sind, die mit einem als Joy-Stick-Körper am proximalen Führungsstabende angebrachten Griffkörper verbunden sind. Durch Verdrehen oder Neigen des Joy-Stick-Körpers erfahren die Zugdrähte entsprechende Längsverschiebungen, deren Auslenkungen zur Verformung der Spitze des Führungsstabes dienen.

Der Druckschrift US 2013/0317300 A1 ist eine Kombination aus einem Videolaryngoskop und einem videogestützten, steuerbaren Führungsstab zu entnehmen, auf den ein Intubationsschlauch geschoben ist. Der Führungsstab überragt distalseitig den Intubationsschlauch mit einem flexibel gestalteten Stabendabschnitt, der räumlich auslenkbar gestaltet ist und die Sichtoptik des videogestützten, steuerbaren Führungsstabs umschließt.

Die Druckschrift US 2009/0050146 A1 offenbart eine Handhabungseinheit zum Platzieren eines ETTs mittels eines innerhalb des ETTs angeordneten Führungsstabes, der den ETT distalseitig überragt. Überdies weist die Handhabungseinheit eine Separiermechanik zum Separieren des Führungsstabes vom ETT auf.

Die Druckschrift WO 2018/109033 A1 beschreibt eine Vorrichtung zum Einführen und Platzieren eines ETTs mittels eines aktiv verformbaren Führungsstabes, dessen aktiv steuerbares Element den ETT distalseitig komplett überragt und der längs zweier Stababschnitte mittels getrennter Seilzüge individuell verformbar ist, um den trachealen Einführvorgang zu erleichtern.

Weitere Intubationsvorrichtungen sind den Druckschriften US 9,888,832 B2, US 10,758,708 B2 sowie WO 2020/247386 A1 zu entnehmen, die allesamt einen steuerbaren Führungsstab besitzen, d.h. der Distalbereich des Führungsstabs ist steuerbar beweglich ausgebildet und muss technisch bedingt stets über das distale Ende des ETT herausragen, wie auch im Falle aller zuvor beschriebenen gattungsgemäßen Einführvorrichtungen. Systembedingt weist der ETT einen größeren Außendurchmesser als der Führungsstab auf. Beim Einführvorgang des ETTs werden somit zuerst der Führungsstab durch die Stimmbandebene in die Luftröhre vorgeschoben und nachfolgend der ETT durch die Stimmbandebene über den bereits in der Luftröhre platzierten Führungsstab nachgeschoben. Durch den Kalibersprung zwischen den Außendurchmessern von Führungsstab und ETT unterliegen die Stimmbänder einem hohen Verletzungsrisiko durch den in der Regel blind vorgeschobenen ETT. Des Weiteren kommt es durch die Kalibiersprünge und durch die unterschiedlichen Winkel (Rachen - Kehlkopf - Luftröhre) vor allem beim Vorschub des ETT über den vorgelegten Führungsstab am Kehlkopf zu mechanischen Hindernissen, d.h. der ETT bleibt beim Vorschub an den Bestandsteilen des Kehlkopfes bzw. an den Stimmbänder hängen und kann nicht ohne weitere Manipulation (Rückzug, Drehungen, erneute Laryngoskopie oder Anwenden von erheblich höheren Schubkräften) vorgeschoben werden. Dies bedeutet wertvolle Zeit, die bei unnötigem Verstreichen zur Sauerstoffunterversorgung und/oder Aspiration, und/oder zu erheblichen Verletzungen führen kann, wie z.B. Stimmbandschäden oder Blutungen. Diese Blutungen können bedingt durch die erheblich schlechter werdende Sicht den Intubationsvorgang gänzlich verhindern, so dass ein Luftröhrenschnitt oder der Tod die Folge sein können.

Zudem muss der Führungsstab bei den derzeit bekannten und im Einsatz befindlichen Führungsstäben, die in den Endotracheal-Tubus platziert werden, entweder von einer zweiten Person im Intubationsvorgang entfernt werden, oder der ETT muss von einer zweiten Person vorgeschoben werden, oder das Laryngoskop muss entfernt werden, um den ETT daraufhin blind vorschieben zu können. Dies resultiert aus der Tatsache, dass die Person, die den Patienten intubiert, in der linken Hand das Laryngoskop und in der rechten Hand den einzuführenden ETT hält. Dadurch sind beide Hände okkupiert und es fehlt in jedem Falle eine dritte Hand.

### Darstellung der Erfindung

Der Erfindung liegt die Aufgabe zugrunde, ein medizinisches Instrument zum Intubieren eines Patienten mit einem einen Hohlkanal einschließenden ETT sowie einen längs des Hohlkanals angeordneten, wenigstens in einem axialen Teilbereich plastisch verformbaren Führungsstab mit einem distalen und einem proximalen Stabende, an dessen distalen Stabende ein bidirektional verformbares Führungselement angebracht ist, mit einem distalen und mit einem proximalen Elementende, dessen proximales Elementende am distalen Stabende gefügt ist und das mit einem Steuermittel in Wirkverbindung steht, derart weiterzubilden, so dass die Handhabung des medizinischen Instruments zur Durchführung der gesamten Intubationsprozedur, d.h. Einführen und Platzieren des ETTs mittels des Führungsstabes sowie Separieren des Führungsstabes vom ETT und Entnehmen desselben, durch die intubierende Person einhändig durchführbar sein soll, so dass zu keinem Zeitpunkt eine dritte Hand benötig wird und infolgedessen auch keine Notwendigkeit für eine zweite, den Intubationsvorgang unterstützende Person besteht.

Zur Lösung der der Erfindung zugrunde liegenden Aufgabe ist ein medizinisches Instrument zum Intubieren eines Patienten (oder Tier) mit den Merkmalen des Oberbegriffes des Anspruches 1 dadurch ausgebildet, dass am proximalen Stabende des Führungsstabes eine in Art eines Handgriffes ausgebildete Bedieneinheit angebracht ist, mit einem mit dem Steuermittel verbundenen Aktuator sowie mit einem lösbar fest mit dem ETT in Wirkverbindung stehenden Schub-Zug-Mechanismus, durch den der ETT längs des Führungsstabes bewegbar ist, dass der Handgriff eine Längserstreckung besitzt, die gegenüber einer dem ETT zuordenbaren Längserstreckung einen Winkel α einschließt, mit 90° ≤ α ≤ 135°, dass der Schub-Zug-Mechanismus und der proximale Bereich des ETTs in Längserstreckung des ETTs teilweise überlappen, und dass beim Intubieren des ETTs samt des im Hohlkanal des ETTs angeordneten Führungsstabes mit dem daran angebrachten bidirektional verformbaren Führungselements das distale Ende des ETTs mit dem distalen Elementende abschließt oder das proximale Elementende das distale Ende des ETTs lediglich kuppenartig überragt, so dass bei Verformung des Führungselementes der ETT längs seines distalen Endbereiches aktiv mitverformt wird.

Mit dem lösungsgemäßen medizinischen Instrument ist erstmals für eine intubierende Person die Möglichkeit geschaffen, nach entsprechend manueller, auf empirischer Erfahrungen basierender Vorformung des Führungsstabes diesen zusammen mit dem ETT, d.h. der Führungsstab ist innerhalb des ETT angeordnet, mit nur einer Hand innerhalb des sich an den Rachen anschließenden Atemwegbereichs durch die Stimmbänder hindurch schonend und zielgerichtet zu navigieren und in einer entsprechenden korrekten Position zu platzieren, wobei der ETT auf diese Art aktiv über den Führungsstab verformt werden kann. So ist das am distalen Stabende bidirektional verformbare Führungselement mit Hilfe des an der Bedieneinheit angebrachten Aktors, vorzugsweise mit Hilfe eines einzigen Fingers, beispielsweise des Daumens, kontrolliert bidirektional verformbar bzw. krümmbar, so dass das distale Elementende je nach Auslenkung entweder in eine Richtung oder in die entsprechende Gegenrichtung auslenkbar ist, jeweils in Abhängigkeit der Patienten-spezifischen Atemwegsgeometrie. Die Längenwahl des ETTs, des Führungsstabes sowie des am Führungsstab distalseitig angebrachten bidirektional verformbaren Führungselements sind derart aufeinander abgestimmt, so dass beim Intubieren bzw. Einführen des ETTs samt des im Inneren des ETTs angeordneten Führungsstabes mit dem daran angebrachten bidirektional verformbaren Führungselements in den Luftweg des Patienten, das distale Ende des ETTs mit dem distalen Elementende abschließt bzw. in dem Sinne weitgehend abschließt, so dass das vorzugsweise kuppenartig gestaltete, proximale Elementende, die ansonsten offen mündende distale ETT-Öffnung domförmig bzw. kuppenartig überragt, d.h. das kuppenartig ausgebildete proximale Elementende ragt axial minimal aus der ETT-Öffnung distalwärts heraus. Auf diese Weise entsteht faktisch kein Kalibersprung zwischen dem distalen Führungsstabende und dem ETT, da beide als Einheit die Stimmbandebene passieren.

Zudem bietet das lösungsgemäß ausgebildete medizinische Instrument für die intubierende Person die Möglichkeit, den ETT über den Führungsstab in die Trachea zu platzieren und diesen anschließend vom Führungsstab zu separieren, ohne dass ein manuelles Umgreifen oder die Hilfestellung einer weiteren Person erforderlich wird. Gleichsam der Aktuatorbedienung ist zum Zwecke eines distalwärtigen Vorschubes des ETT über den Führungsstab in die Luftröhre ein an der Bedieneinheit angebrachter Schub-Zug-Mechanismus vorgesehen, der gleichsam der vorstehend erläuterten Aktuatorbedienung auch mit Hilfe nur eines Fingers, vorzugsweise mit Hilfe des Zeigefingers, bedienbar ist.

Die Einhandbedienung des lösungsgemäßen, medizinischen Instrumentes ist besonders geeignet für medizinische Notfalleinsätze, bei denen eine zweite Person fehlt oder anderweitig gebunden ist. Durch eine ergonomische Ausbildung der Bedieneinheit mit den daran angebrachten manuell betätigbaren Aktuator und Schub-Zug-Mechanismus sind instrumentelle Voraussetzungen für ein schnelles und schonendes Intubieren geschaffen, wodurch ein Unterbrechen bzw. Abbrechen von Intubationsvorgängen aufgrund von Aspiration und/oder SauerstoffMinderversorgung ausgeschlossen oder zumindest signifikant reduziert werden können.

Das "einhändige" Bedienkonzept des lösungsgemäßen medizinischen Instruments zum Intubieren eröffnet zudem die Möglichkeit eines Robotereinsatzes, der insbesondere in Pandemie-Zeiten, beispielsweise bedingt durch Corona-Viren, wie (SARS-CoV-2, SARS, MERS) oder Influenza, Ebola, von großem Interesse ist, um die intubierenden Personen zu schützen. Hierzu gilt es die Bedieneinheit in geeigneter Weise in Form eines mechanischen oder mechatronischen Verbindungsflansches zum Zwecke der Adaption an und Betätigung durch eine Robotereinheit auszubilden.

In einer bevorzugten Ausführungsform des medizinischen Instrumentes weist das einerseits mit dem Aktuator und andererseits mit dem distalen Elementende des bidirektional verformbaren Führungselementes verbundene Steuermittel wenigstens zwei Zugkräfte übertragende, vorzugsweise seil- oder bandförmig ausgebildete Strangabschnitte auf, die mit dem Aktuator derart in Wirkverbindung stehen, so dass bei Betätigung des Aktuators beide Strangabschnitte entgegengesetzt zueinander kinematisch auslenkbar sind. Hierzu weist der Aktuator vorzugsweise ein um eine Drehachse drehbar gelagertes Drehelement auf, an dem jeweils die Strangendabschnitte beider Strangabschnitte in zur Drehachse gegenüber liegenden Halbebenen mit dem Drehelement in Wirkverbindung stehen. Die distalseitigen Strangendabschnitte der zwei Zugkräfte übertragenden Strangabschnitte sind jeweils am distalen Elementende des vorzugsweise aus biegeelastischem Material gefertigten Führungselementes an zwei zur Längsachse des biegeelastischen Führungselementes gegenüberliegenden Befestigungsstellen angebracht.

Beide Strangabschnitte sind vorzugsweise gleich lang dimensioniert und nehmen in einer Neutralstellung, in der das vorzugsweise länglich biegeelastisch ausgebildete Führungselement eine geradlinige, d.h. krümmungsfreie Form einnimmt, jeweils einen weitgehendgespannten und Zugkräfte-freien Zustand ein. Bei Betätigung des Aktors, d.h. bei Rotation des Drehelementes, wird einer der beiden Strangabschnitte unter Ausbildung einer auf das Führungselement wirkenden Zugkraft proximalwärts um eine Wegstrecke ausgelenkt, längs der gegenüber liegende Strangabschnitt distalwärts geführt wird. Im Ergebnis krümmt sich das biegeelastische Führungselement in Richtung des Zugkraft-beaufschlagten Strangabschnittes. In der gleichen Weise erfolgt eine Krümmung des Führungselementes in die exakt entgegengesetzt orientierte Richtung, bei Betätigung des Aktors und Drehung des Drehelementes in entgegengesetzter Richtung um die Drehachse.

In einer weiteren Ausführungsform sind die mit dem Drehelement in Wirkverbindung stehenden Strangendabschnitte beider Strangabschnitte einstückig bzw. monolithisch miteinander verbunden. Das Drehelement ist in diesem Fall in Art einer Drehscheibe mit einem nutförmig ausgebildeten Umfangsrand ausgebildet, längs dessen Umfangsrandhälfte das seil- oder bandförmig ausgebildete Steuermittel enganliegend umläuft.

Alternativ zur Ausbildung des Steuermittels in Art einer mechanischen Bowdenzug-Lösung, die mit dem biegeelastisch ausgebildeten Führungselement zusammenwirkt, wie vorstehend beschrieben, bietet es sich ebenso an ein geeignet konfektioniertes, biegeelastisches Führungselement mittels elektrischer, pneumatischer oder hydraulischer Steuersignale kontrolliert zu krümmen bzw. zu verformen. Hierzu weist das bidirektional verformbare Führungselement eine Baueinheit auf, die durch elektrische, pneumatische oder hydraulische Steuersignale eine Formänderung des Führungselementes zu initiieren vermag.

In einer Ausführungsvariante weist die Baueinheit wenigstens eine der nachfolgenden, mittels elektrischer Steuersignale aktivierbare elektrische bzw. mechatronische Komponenten auf: elektromotorische Getriebeeinheit, Aktor-Sensor-Einheit, piezoelektrischer Werkstoff, magnetostriktiver Werkstoff, Formgedächtnislegierung, etc.. Beispielsweise im Falle der Verwendung von piezioelektrischen Materials, das als integraler Bestandteil an, längs und/oder innerhalb des Führungselementes vorgesehen ist, führen elektrisch initiierte Materialkontraktionen zu Formänderungen des Führungselementes. Die elektrischen Steuersignale sind durch einen elektrischen Schaltkreis, wenigstens enthaltend eine elektr. Energiequelle sowie eine Schaltereinheit, an das piezoelektrische Material applizierbar. Vorzugsweise sind die elektr. Energiequelle sowie die als Aktuator dienende Schaltereinheit in der Bedieneinheit untergebracht. Längs des Führungsstabes verlaufen lediglich zwei elektrische Leitungswege, um die jeweils im Führungselement zum Zwecke dessen Formänderung integrierte elektrische Komponente anzusteuern.

In gleicher Weise sind in alternativen Ausführungsbeispielen innerhalb des Führungselementes integrierte pneumatisch oder hydraulisch aktivierbare Komponenten, bspw. in Form wenigstens eines Bläh- oder Füllkörpers bzw. -volumens, mittels längs des Führungsstabes verlaufenden Fluidleitungen ansteuerbar. In diesen Fällen enthält der hierzu geeignet konfektionierte pneumatische oder hydraulische Schaltkreislauf bzw. Fluidkreislauf über eine Pumpeneinheit sowie ein als Aktuator dienendes Steuerventil, die in geeigneter Weise Teil der Bedieneinheit sind.

Zum Zwecke einer möglichst komfortablen und ermüdungsfreien Handhabung des medizinischen Instrumentes ist die als Handgriff ausgebildete Bedieneinheit in Art eines ergonomisch geformten Umgreifungskörpers ausgebildet, der einseitig vom Daumen und gegenüber liegend von der übrigen Hand umschlossen werden kann. Zur Betätigung des Aktuators sieht dieser wenigstens ein erstes Bedienelement vor, das derart am Umgreifungskörper ergonomisch angeordnet und ausgebildet ist, dass das erste Bedienelement mittels des Daumens einer den Umgreifungskörper umschließenden Hand betätigbar ist. Hierzu ist das erste Bedienelement vorzugsweise in Art eines Hebels oder Stellrades ausgebildet, das vom Daumen entweder in die eine oder die entgegengesetzte Richtung schwenkbar bzw. verstellbar ist.

Darüber hinaus ist am ergonomisch ausgebildeten Handgriff ein zweites Bedienelement derart ergonomisch angeordnet und ausgebildet, dass das zweite Bedienelement mittels des Zeige- bzw. Mittelfingers der den Handgriff umschließenden Hand betätigbar ist. Das zweite Bedienelement dient zur Betätigung des Schub-Zug-Mechanismus, durch den der ETT längs des Führungsstabes bewegbar ist. Das zweite Bedienelement ist hierzu vorzugsweise in Art eines am Handgriff angebrachten Hebels ausgebildet, der vermittels des Zeige- bzw. Mittelfingers sowohl in Richtung des Handgriffes gezogen werden kann, als auch in entgegengesetzter Richtung vom Handgriff weggedrückt werden kann. Vorzugsweise ist das zweite Bedienelement ringförmig ausgebildet, durch das der Zeige- bzw. Mittelfinger hindurch gesteckt werden kann.

Das in der vorstehenden Weise ausgebildete zweite Bedienelement ist über eine im Handgriff angeordnete Getriebeeinheit mit einer Schub-Zug-Stange kinematisch verbunden, die lösbar fest mit dem proximalen Bereich des ETT gefügt ist. Die Getriebeeinheit vermag bei proximalwärtiger Auslenkung des zweiten Bedienelementes in Richtung des Handgriffes die Schub-Zug-Stange sowie den mit dieser lösbar fest gefügten ETT distalwärts und bei entgegengesetzter Auslenkung des zweiten Bedienelementes die Schub-Zug-Stange sowie den mit dieser lösbar fest gefügten ETT proximalwärts auszulenken bzw. zu schieben. Vorzugsweise sieht die Getriebeeinheit ein um eine Drehachse drehbar gelagertes Zahnrad mit Außenverzahnung vor, in die sowohl eine mit dem zweiten Bedienelement verbundene Zahnstange als auch die in Art einer Zahnstange ausgebildete Schub-Zug-Stange eingreifen. Eine bevorzugte Ausführungsform zur konstruktiven Ausgestaltung der Getriebeeinheit ist nachstehend unter Bezugnahme auf die Figuren detailliert illustriert und beschrieben.

Der bereichsweise plastisch und flexibel verformbare Führungsstab weist eine Länge auf, die in etwa der Länge des ETT's entspricht. Aufgrund der Material-inhärenten Flexibilität des ETT's um dessen Längsachse ist es vorteilhaft, jedoch nicht erforderlich, dass das biegeelastische am proximalen Ende des Führungsstabes angebrachte Führungselement distalseitig höchstens minimal über den ETT ragt.

In einer weiteren Ausführungsform ist längs des Lumens durch den Führungsstab, durch das die Strangabschnitte des Steuermittels geführt sind, oder eines weiteren Lumens durch den Führungsstab ein Beobachtungsmittels geführt, das beispielsweise in Form eines Beleuchtungs-Katheters, eines Kamera-Katheters oder eines LIDAR-Sensor unterstützten Katheters ausgebildet sein kann. Auf diese Weise ist es möglich, den Intubationsvorgang visuell überwacht durchzuführen und die kontrolliert vorgebbare Krümmung des Führungselementes in situ individuell an die räumlichen Atemwegs-Gegebenheiten anzupassen, um den ETT schonend und schnellstmöglich zu platzieren.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnung exemplarisch beschrieben. Es zeigen:
- Fig. 1: Ausführungsbeispiel eines erfindungsgemäßen medizinischen Instrumentes zum Intubieren eines Patienten,
- Fig. 2: Führungsstab mit bidirektional verformbaren Führungselement,
- Fig. 3: Detaildarstellung des verformbaren Führungselementes sowie
- Fig. 4: Führungsstab mit bidirektional verformbaren Führungselement mit elektrisch, pneumatisch oder hydraulisch aktivierbarer Baueinheit.

### Wege zur Ausführung der Erfindung, gewerbliche Verwendbarkeit

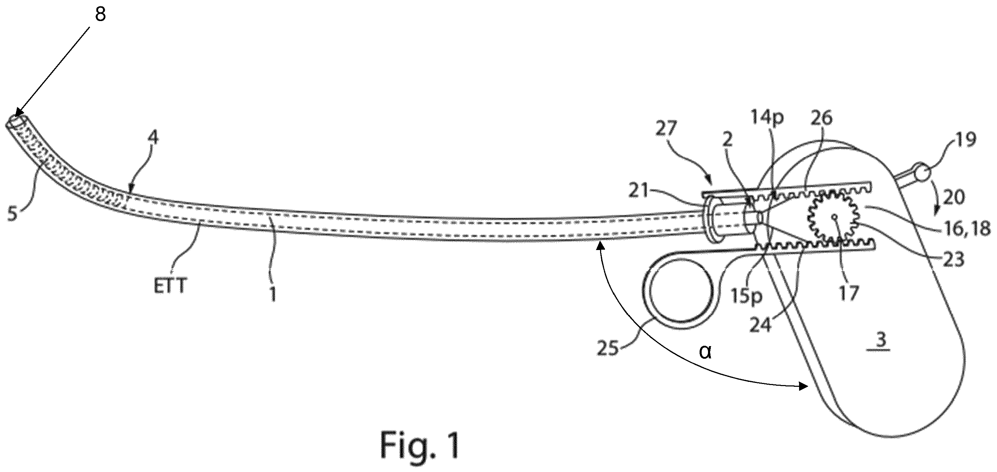

In Figur 1 ist eine schematisierte Gesamtdarstellung des medizinischen Instrumentes zum Intubieren eines Patienten mit ETT illustriert, das einen Führungsstab 1 aufweist, dessen proximales Stabende 2 mit einer als Handgriff ausgebildeten Bedieneinheit 3 verbunden ist und an dessen distalen Stabende 4 ein bidirektional verformbares Führungselement 5 angeordnet ist.

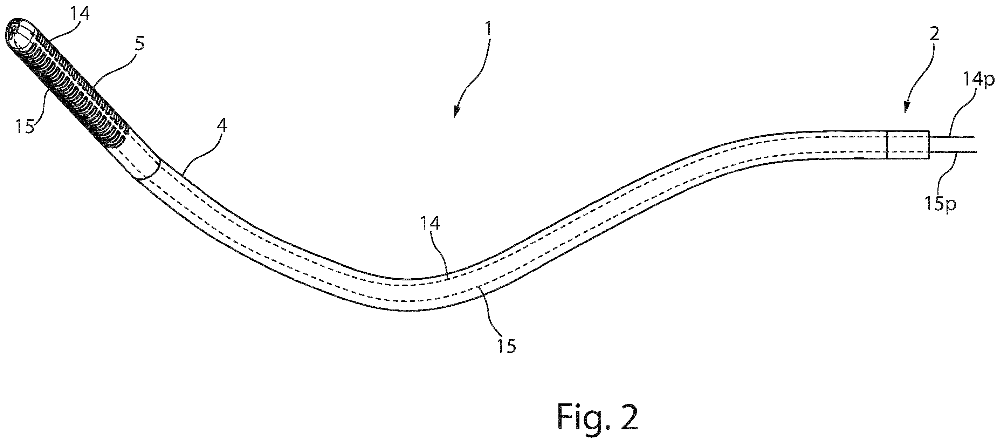

In Figur 2 ist der Führungsstab 1 sowie das an dessen distalen Stabende 4 angeordnete bidirektional verformbare Führungselement 5 in Alleindarstellung illustriert.

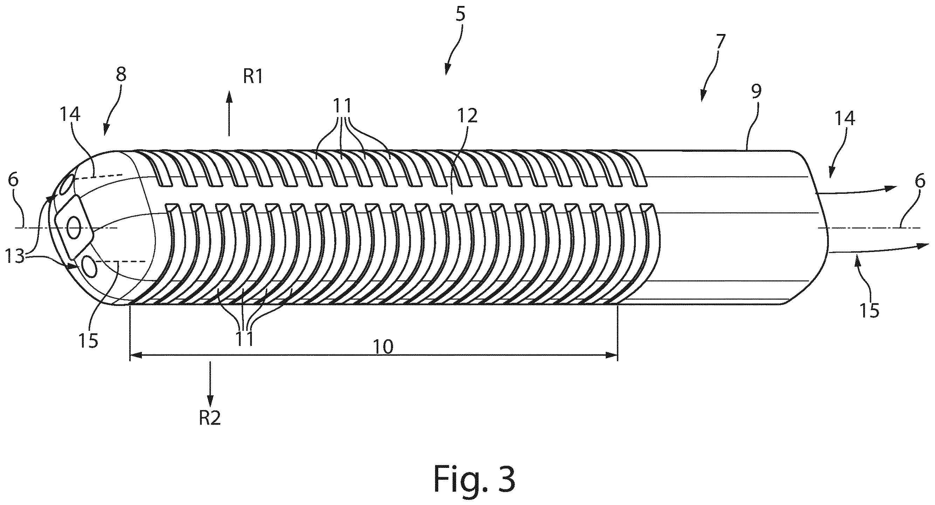

Figur 3 zeigt eine bevorzugte Ausführungsform eines bidirektional verformbaren Führungselementes 5 in Detailansicht. Die weiteren Ausführungen nehmen gemeinsamen Bezug auf die Figuren 1 bis 3.

Der als Hohlkanal ausgebildete Führungsstab 1 ist vorzugsweise aus einem plastisch verformbaren Material gefertigt, so dass eine intubierende Person den Führungsstab 1 zu Zwecken einer möglichst patientenschonenden oralen Einführung in den Rachenbereich sowie den daran anschließenden Atemweg manuell vorzukrümmen vermag, wie dies in Figur 2 dargestellt ist. Alternativ zur Ausbildung des Führungsstabes 1 als Hohlkanal bietet es sich an, den Führungsstab 1 in Form einer Kreuzkammerkonstruktion auszubilden, d.h. der Führungsstab 1 weist einen kreuzförmigen Querschnitt auf und ist unmittelbar vom ETT umgeben. Durch die längs innerhalb des ETTs verlaufenden Kreuzkammern können die Zugdrähte relativ widerstandslos laufen, wobei der kreuzartige Querschnitt des Führungsstabes eine Führung der Zugdrähte sowie die nötige physikalische Steifigkeit ermöglicht.

Das am distalen Stabende 4 gefügte bidirektional verformbare Führungselement 5 besteht gemäß dem in Figur 3 im Detail dargestellten Ausführungsbeispiel aus einem monolithisch gefertigten hülsenartig ausgebildeten Hohlkörper, dem eine Längsachse 6, ein proximales Elementende 7 sowie ein distales Elementende 8 zugeordnet ist. Das proximale Elementende 7 ist einseitig fest mit dem distalen Stabende 4 des Führungsstabes 1 gefügt. Das distale Elementende 8 ist kuppenartig ausgebildet und verfügt über eine weitgehend geschlossene Oberfläche, um ein möglichst läsionsfreies und gleitendes Eindringen des Führungsstabes 1 in den Atemwegsbereich bzw. zum einfach Einführen des Führungsstabes in den ETT zu gewährleisten. Die axiale Länge des ETTs ist derart bemessen, dass während des Einführens des ETTs in den Atemweg eines Patienten mittels des medizinischen Instrumentes, der ETT den Führungsstab 1 sowie das an diesem bidirektional verformbar angebrachte Führungselement 5 in axialer Erstreckung vollständig umgibt. In vorteilhafter Weise ragt lediglich das kuppenartig gestaltete distale Elementende 8 des Führungselementes 5 axial durch bzw. über das distale Ende des ETTs, um die intrakorporale Vorschubbewegung durch Stimmband- und Luftröhren mit der Kuppenform so -schonend wie möglich zu gestalten, wie dies auch aus Figur 1 zu entnehmen ist.

Der ETT ist aus einem Material mit einer Material-inhärenten Flexibilität gefertigt, wodurch der ETT biegsam um dessen Längsachse ist und so einer durch eine plastische Vorbiegung des Führungsstabes 1 und einer gesteuerten Auslenkung des Führungselementes 5 vorgegebenen Raumform zu folgen vermag. Ein Vorteil des medizinischen Instrumentes rührt von der aktiven räumlichen Verformbarkeit des distalen Bereiches des ETTs her, die durch das Führungselement 5 initiiert wird und für einen direkten intrakorporalen Vorschub des ETTs längs des Atemweges ermöglicht. Dabei kann der der Großteil des ETT vermittels des plastisch verformbaren Anteils des Führungsstabes vorgeformt werden unter Maßgabe des Patienten-spezifischen Atemwegsverlaufes, wobei der distale Endbereich des ETTs zu Navigationszwecken beweglich bleibt. Dies führt zu einem erheblich schnelleren Ablauf vom Beginn der Intubation bis zur Möglichkeit der Beatmung des Patienten. Auf diese Weise können schwerwiegende Schäden am Patienten durch etwaigen Sauerstoffmangel vermieden werden.

Ein weiterer Vorteil ergibt sich aus der axialen Lagekoinzidenz der distalen Enden des ETTs und des bidirektional verformbaren Führungselementes 5, wodurch ein die Stimmbänder irritierender Kalibersprung, wie er bei bekannten gattungsgemäßen Intubiervorrichtungen mit einem dem ETT vorauseilenden Führungsstab 5 unvermeidbar ist, nicht auftritt. So ist systembedingt der Durchmesser des Führungsstabes stets kleiner als der endgültig zu platzierende ETT. In der Praxis bedeutet dies, dass bei einem dem ETT vorauseilenden Führungsstab dieser in den meisten Fällen problemlos in der Luftröhre platziert werden kann, jedoch kann der nachfolgende ETT aufgrund des unsteten größeren Außendurchmessers die Stimmlippen bzw. Stimmbänder häufig nicht oder nur unter Kraftaufwand, bzw. Rotationsbewegungen an den Stimmbändern vorbei geschoben werden, d.h. eine Verletzung derselben kann die Folge sein. Dies kann zu Heiserkeit ggf. für den Rest des Lebens führen. Des Weiteren kann z.B. und v.a. bei Notfallpatienten durch die erhöhte Kraftausübung umgebendes Gewebe verletzt werden. Daraus resultieren nicht selten akute Blutungen, die die Sicht und dadurch die Intubation unmöglich machen können. Die Folge wäre ein Luftröhrenschnitt oder wenn dies nicht gelingt der Tot des Patienten.

Durch das lösungsgemäße, aufeinander abgestimmte Längenmaß von ETT sowie dem darin geführten Führungsstab samt Führungselement schließt das distale Ende des ETTs mit dem distalen Elementende des Führungselementes ab oder das proximale Elementende überagt das distale Ende des ETTs lediglich kuppenartig und der ETT kann bei entsprechender auf den Patienten abgestimmten Querschnittsdimensionierung wie bei einer Intubation ohne Führungsstab unter Sicht direkt durch die Stimmlippen platziert werden. Auf diese Weise vermag ein Bediener den ETT atemwegsschonend durch die Stimmbandebene hindurch zu führen und das distale ETT-Ende in Intubationsrichtung hinter den Stimmbändern schnell und sicher zu platzieren.

Das in Figur 3 illustrierte Führungselement 5 stellt einen zur Längsachse 6 symmetrischen Hohlkörper dar. Im Bereich des proximalen Elementendes 7 weist das Führungselement 5 zu Zwecken einer möglichst formstabilen Fügung an den Führungsstab 1 eine unterbrechungsfreie Hülsenwand 9 auf, an die sich distalwärts ein bidirektional verformbarer Abschnitt 10 anschließt. In diesem Abschnitt 10 ist die Hülsenwand strukturiert und weist jeweils orthogonal zur Längsachse 6 durch Abstände axial voneinander getrennte rippenbogenartig ausgebildete Wandabschnitte 11 auf, die jeweils beidseitig mit einem Hülsenwandsteg 12, der sich jeweils von der proximalseitig unterbrechungsfreien Hülsenwand 9 bis zum distalen Elementende 8 erstreckt, verbunden sind. Der Ordnung halber sei darauf hingewiesen, dass gegenüber dem in Figur 3 sichtbaren Hülsenwandsteg 12 ein entsprechender Hülsenwandsteg an der nicht in Figur 3 sichtbaren Rückseite des Führungselementes 5 angeordnet ist.

Der in der vorstehenden Weise strukturierte und in Figur 3 dargestellte, bidirektional verformbare Abschnitt 10 ermöglicht eine Krümmung des Führungselementes 5 in die beiden, durch Pfeile dargestellten, entgegengesetzt zueinander orientierten Richtungen R1, R2, die jeweils quer zur Längsachse 6 orientiert sind.

Zur kontrollierten Auslenkung des bidirektional verformbaren Führungselementes 5 jeweils längs eines der beiden Krümmungsrichtungen R1, R2 ist am distalen Elementende 8 eine Befestigungsstruktur 13 zur Fixierung der jeweils distalen Elementenden, der zwei Zugkräfte übertragenden Strangabschnitte 14, 15 angebracht. Die Strangabschnitte 14, 15 sind seil- oder bandförmig ausgebildet und verlaufen im Inneren des als Hohlkörper ausgebildeten Führungselementes 5 proximalwärts durch den sich proximalwärts zum Führungselement 5 anschließenden, als Hohlkanal ausgebildeten Führungsstab 1, siehe auch Figur 2.

Die proximalen Strangendabschnitte 14p, 15p der Strangabschnitte 14, 15, siehe Figur 1, ragen proximalseitig aus dem Führungsstab 1 und sind mit einem in der als Handgriff ausgebildeten Bedieneinheit 3 integrierten Aktuator 16 verbunden. Der in Figur 1 illustrierte Aktuator 16 weist ein um eine Drehachse 17 drehbar gelagertes Drehelement 18 auf, an dem ein Bedienhebel 19 angelenkt ist, der mittels des Daumens einer den Handgriff 3 umschließenden Hand bedienbar ist. Bei Schwenken des Bedienhebels 19 in die in Figur 1 dargestellte Schwenkrichtung 20 wird der obere Strangabschnitt 14 proximalwärts gezogen, wohingegen der untere Strangabschnitt 15 distalwärts geführt wird. In diesem Fall wirkt längs des oberen Strangabschnittes 14 eine Zugkraft, die das distalseitig am Führungsstab 1 angebrachte Führungselement 5 in der in Figur 1 dargestellten Weise zu krümmen vermag. Bei Auslenkung des Bedienhebels 19 in die zur Schwenkrichtung 20 entgegengesetzte Schwenkrichtung erfolgt eine entsprechende Krümmungsumkehr des Führungselementes 5.

In Figur 1 ist über den Führungsstab 1 ein Endotracheal-Tubus, kurz ETT, geschoben, an dessen proximalen Ende ein ringförmiger Konnektor 21 angebracht ist, der Bestandteil des ETTs ist. Der Konnektor 21 steht in Wirkverbindung mit einem im Handgriff 3 ebenfalls integrierten Schub-Zug-Mechanismus 22, der durch manuelle Betätigung den ETT längs des Führungsstabes 1 auszulenken vermag. Der Schub-Zug-Mechanismus 22 weist ein um die Drehachse 17 drehbar gelagertes Zahnrad 23 mit Außenverzahnung auf, in die einerseits eine Zahnstange 24 eingreift, die einseitig als ringförmiges, zweites Bedienelement 25 ausgebildet ist, in die der Zeigefinger einer den Handgriff 3 umfassenden Hand eingreifen kann. Zum anderen greift eine weitere Zahnstange 26 gegenüberliegend zur Zahnstange 24 in die Außenverzahnung des Zahnrades 23 ein, die endseitig über einen Rastmechanismus 27 mit dem Kragen 21 des ETTs in lösbar fester Wirkverbindung steht.

Durch proximalwärtige Auslenkung der Zahnstange 24 wird in gleichem Maße die Zahnstange 26 und der mit dieser verbundene ETT distalwärts geschoben.

Gleichsam ist es möglich, durch distalwärtige Auslenkung der Zahnstange 24 vermittels des zweiten Bedienelementes 25 den ETT proximalwärts längs des Führungsstabs 1 zu bewegen.

Somit ist es möglich, mit nur einer Hand das medizinische Instrument mit vorgeformtem Führungsstab 1 in den Atemweg einer Person einzuführen und durch geeignete Betätigung des ersten Bedienhebels 19, vorzugsweise mit dem Daumen, das bidirektional verformbare Führungselement 5 zu Zwecken einer patientenschonenden Instrumenteneinführung und erleichterten Navigation individuell bidirektional zu krümmen, wodurch auch der ETT längs seiner das Führungselement 5 umschließenden Erstreckung mitgekrümmt wird und so synchron zum Führungsstab 5 intrakorporal platziert werden kann. Nach entsprechender Platzierung kann sodann der ETT vermittels des zweiten Bedienelementes 25, das in geeigneter Weise mit dem Zeigefinger betätigbar ist, distalwärts in die Trachea vorgeschoben werden. Nach erfolgter Platzierung und Fixierung des ETTs, bspw. in an sich bekannter Weise mit einem am ETT angebrachten inflatierbaren Ballon (sog. Cuff, nicht dargestellt), wird der Rastmechanismus 27 von dem fest mit dem ETT verbundenen Kragen 21 gelöst, und der Führungsstab 1 proximalwärts aus dem ETT gezogen.

Auch und insbesondere die Ausformung und Anbringung des Handgriffes 3 in Art eines "Pistolen"-Griffes relativ zu dem einhändig handzuhabenden ETT und dem am Führungsstab 1 angebrachten bidirektional verformbaren Führungselement 5 tragen zu einer ergonomisch günstigen und für den Intubationsvorgang krafteffizienten Handhabung bei. So ist es möglich während des gesamten Intubationsvorgang die ETT-Spitze bidirektional zu verformen und in einer bestimmten Form zu halten. Dies wird dadurch erzielt, indem die den Handgriff 3 umfassende Hand und somit die dem Handgriff 3 zuordenbare Längserstreckung weitgehend orthogonal zur Vorschubrichtung längs des ETTs orientiert ist. Hierdurch wird zum einen eine effizientere Kraftübertragung und zum anderen können größere Auslenkwinkel um die Längsachse des ETTs, von bis zu 120°, erzielt werden. Typischerweise ist die Längserstreckung des Handgriffes 3 gegenüber der Längserstreckung des ETTs um einen Winkel α geneigt, mit 90° ≤ α ≤ 135° Durch diese weitgehende orthogonale Ausrichtung sitzt der Schub-Zug-Mechanismus 22 annähernd auf der Höhe des Rastmechanismus 27, der die Verbindung zum ETT herstellt, wodurch eine Unmittelbarkeit in der Handhabung des Bedienelementes 25 und der hierdurch resultierenden ETT-Bewegung mit einer damit zusammenhängenden präzisen Handhabung des ETTs bewirkt wird.

Da der Führungsstab 5 proximalseitig in etwa auf der Höhe des ETTs endet und der Handgriff 3 direkt am proximalen ETT-Ende anschließt, ist eine kompakte Baulänge der medizinischen Vorrichtung möglich. Dadurch werden z.B. Seitwärtsbewegungen sehr direkt auf den ETT übertragen, zudem treten nur geringe Verwindungs- bzw. Scherkraftverluste auf. Durch die direkte Kraftübertragung erhält der Bediener eine exakte haptische Kontrolle, d.h. das Prozedere der Einführung des Beatmungsschlauches kann schneller und sicherer vollzogen werden und einwirkenden Kräfte werden besser rückgekoppelt, wodurch z.B. Stimmbandschäden oder gar eine Durchspießung der Luftröhre unwahrscheinlicher wird. Durch das kompakte Design der lösungsgemäßen medizinischen Vorrichtung eignet sich diese bei entsprechender Dimensionierung z.B. auch erstmals für Säuglingsintubationen, da in diesen Fällen eine sehr feine haptische Kontrolle essentiell ist.

Durch die Ausbildung und Anbringung des Handgriffes 3 können einhändig sechs Freiheitsgrade an der distalen ETT-Spitze erreicht werden, d.h. bidirektionale aktive Biegung durch den ersten Bedienhebel 19, Rechts-/Links-Rotation durch Rotation des gesamten Handgriffes, sowie eine axiale Vor- und Zurückbewegung des gesamten ETT durch das zweite Bedienelement 25. Hierdurch kann der optimale Winkel für die ETT-Spitze für die Passierung der Stimmbänder eingestellt werden und nach der Passierung der Stimmbänder weiter in der anatomisch vorgegebenen Richtung bewegt werden, um den ETT hinter den Stimmbändern in die axiale Richtung der Luftröhre zu krümmen.

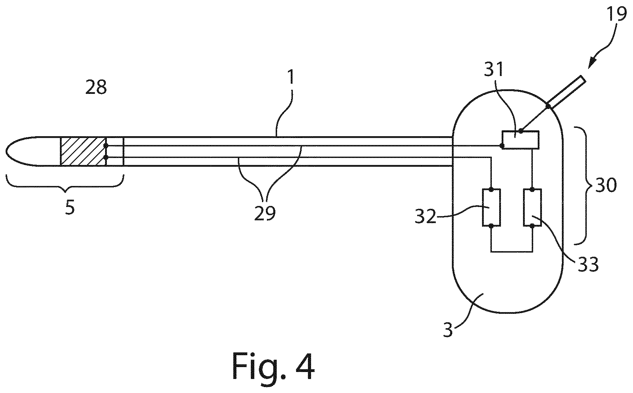

Figur 4 illustriert einen Führungsstab 1 mit einer proximalseitig angebrachten, als Handgriff ausgebildeten Bedieneinheit 3. Distalseitig am Führungsstab 1 ist ein elastisch biegsames Führungselement 5 vorgesehen, das über eine Baueinheit 28 als integrale Komponente verfügt, die durch elektrische, pneumatische oder hydraulische Steuersignale eine Formänderung des Führungselementes 5 zu initiieren vermag. Zur weiteren Erläuterung sei angenommen, dass die Baueinheit 28 elektrisch betreibbar und aktivierbar ist und in Form einer der nachfolgenden Komponenten ausgebildet ist: elektromotorische Getriebeeinheit, Aktor-Sensor-Einheit, piezoelektrischer Werkstoff, magnetostriktiver Werkstoff, Formgedächtnislegierung, etc..

Die Baueinheit 28 ist über zwei elektrische Leitungswege 29, die längs des Führungsstabes 1 angeordnet sind, mit einem in der Bedieneinheit 3 angeordneten elektrischen Schaltkreis 30 verbunden. Der elektrische Schaltkreis 30 enthält im dargestellten Fall, eine elektrische Energiequelle 31, eine Schaltereinheit 32, die als Aktuator dient und durch den ersten Bedienhebel 19 betätigbar ist, sowie eine elektrische Steuer- und Kontrolleinheit 33.

Anstelle der in Figur 4 illustrierten, als Handgriff ausgebildeten Bedieneinheit 3 ist es ebenso denkbar die Bedieneinheit als Verbindungsflansch zur Adaption an und Betätigung durch eine Robotereinheit auszubilden. In diesem Fall erfolgt die Ansteuerung der im Führungselement 5 integrierten Baueinheit 28 durch ein roboterseitig vorgesehenes Steuersystem, das zugleich die Robotermotorik kontrolliert, um auf diese Weise die Voraussetzungen für ein autonomes Intubieren zu schaffen. Dies setzt selbstverständlich voraus, dass am distalen Bereich des Führungselementes eine geeignete Sensorik angebracht ist. So bietet es sich bspw. an, wie vorstehend bereits erwähnt, durch das Lumen des Führungsstabes oder eines weiteren Lumens durch den Führungsstab ein Beobachtungsmittel zu führen, beispielsweise in Form eines Beleuchtungs-Katheters, eines Kamera-Katheters und/oder eines LIDAR-Sensor unterstützten Katheters.

Auf die Darstellung der zweiten Bedieneinheit 25 sowie des Schub-Zug-Mechanismus 22 an der als Handgriff ausgebildeten Bedieneinheit 3 ist in Figur 4 aus Gründen einer übersichtlichen Darstellung verzichtet worden.

Insgesamt ist festzuhalten, dass die schnelle und richtige Platzierung des ETTs eine eminent lebenswichtige Rolle bei der Narkoseeinleitung,in der Notfallversorgung sowie der Intensivmedizin spielt, wobei Probleme sehr schnell zu lebensbedrohlichen Komplikationen führen können. Im Falle eines bekannt oder unbekannt schwierigen Atemweges kommen verschiedenste Methoden und Hilfsmittel zum Einsatz. Speziell besteht das Bedürfnis sowohl den ETT per innenliegendem passivem Führungsstab gezielt in eine anatomische Form zu bringen, als auch die ETT-Spitze aktiv zu steuern, um so verletzungsfrei wie möglich die Stimmbandebene zu passieren und danach in axialer Luftröhrenrichtung zu platzieren. Die lösungsgemäße medizinische Vorrichtung ermöglicht erstmals die vorstehenden Maßnahmen ohne der Notwendigkeit der Assistenz durch eine zweite Person.

### Bezugszeichenliste

- 1: Führungsstab
- 2: proximales Stabende
- 3: Bedieneinheit, Handgriff
- 4: distales Stabende
- 5: bidirektional verformbares Führungselement
- 6: Längsachse
- 7: proximales Elementende
- 8: distales Elementende
- 9: Unterbrechungs-freie Hülsenwand
- 10: bidirektional verformbarer Abschnitt
- 11: rippenbogenartige Wandabschnitte
- 12: Hülsenwandsteg
- 13: Befestigungsstruktur
- 14,15: Strangabschnitt
- 14p, 15p: Strangabschnittsende
- 16: Aktor
- 17: Drehachse
- 18: Drehelement
- 19: erster Bedienhebel
- 20: Schwenkrichtung
- 21: Konnektor
- 22: Schub-Zug-Mechanismus
- 23: Zahnrad
- 24: Zahnstange
- 25: zweites Bedienelement
- 26: Zahnstange
- 27: Rastmechanismus
- 28: Baueinheit
- 29: elektrische Leitungswege
- 30: elektrischer Schaltkreislauf
- 31: elektrische Energiequelle
- 32: Schaltereinheit
- 33: Steuer- und Kontrolleinheit
- R1, R2: Krümmungsrichtungen
- α: Neigungswinkel der Längsrichtung der Bedieneinheit relativ zur ETT-Längserstreckung

## Patentansprüche

1. Medizinisches Instrument zum Intubieren eines Patienten mit einem einen Hohlkanal einschließenden Endotrachealtubus, kurz ETT, sowie einem längs des Hohlkanals angeordneten, wenigstens in einem axialen Teilbereich plastisch verformbaren Führungsstab (1) mit einem distalen (4) und proximalen (2) Stabende, an dessen distalen Stabende ein bidirektional verformbares Führungselement (5) angebracht ist mit einem distalen und einem proximalen Elementende, dessen proximales Elementende am distalen Stabende gefügt ist, und das mit einem Steuermittel in Wirkverbindung steht,
wobei am proximalen Stabende des Führungsstabes eine in Art eines Handgriffes ausgebildete Bedieneinheit (3) angebracht ist, mit einem mit dem Steuermittel verbundenen Aktuator (16) sowie mit einem lösbar fest mit dem ETT in Wirkverbindung stehenden Schub-Zug-Mechanismus, durch den der ETT längs des Führungsstabes bewegbar ist,
dass der Handgriff eine Längserstreckung besitzt, die gegenüber einer dem ETT zuordenbaren Längserstreckung einen Winkel α einschließt, mit 90° ≤ α ≤ 135°, dass der Schub-Zug-Mechanismus und der proximale Bereich des ETTs in Längserstreckung des ETTs teilweise überlappen, und
dass beim Intubieren des ETTs samt des im Hohlkanal des ETTs angeordneten Führungsstabes mit dem daran angebrachten bidirektional verformbaren Führungselements das distale Ende des ETTs mit dem distalen Elementende abschließt oder das proximale Elementende das distale Ende des ETTs lediglich kuppenartig überragt, so dass bei Verformung des Führungselementes der ETT längs seines distalen Endbereiches aktiv mitverformbar ist.

2. Medizinisches Instrument nach Anspruch 1,
wobei das Steuermittel wenigstens zwei Zugkräfte übertragende Strangabschnitte aufweist, mit jeweils einem am distalen Elementende des bidirektional verformbaren Führungselementes fixierten Strangabschnittsende sowie jeweils einem dem am Führungselement fixierten Strangabschnittende gegenüberliegenden Strangendabschnitt, der mit dem Aktuator derart in Wirkverbindung steht oder bringbar ist, dass bei Betätigung des Aktuators beide Strangabschnitte entgegengesetzt zueinander kinematisch auslenkbar sind.

3. Medizinisches Instrument nach Anspruch 2,
wobei der Aktuator ein um eine Drehachse drehbar gelagertes Drehelement aufweist, an dem die Strangendabschnitte beider Strangabschnitte in jeweils zur Drehachse gegenüberliegenden Halbebenen mit dem Drehelement in Wirkverbindung stehen.

4. Medizinische Instrument nach Anspruch 2 oder 3,
wobei die zwei Zugkräfte übertragenden Strangabschnitte an den Strangendabschnitten einstückig miteinander verbunden sind.

5. Medizinisches Instrument nach einem der Ansprüche 1 bis 4,
wobei der Handgriff einen ergonomisch geformten Umgreifungskörper aufweist,
dass der Aktuator wenigstens ein erstes Bedienelement vorsieht, das am Umgreifungskörper derart ergonomisch angeordnet und ausgebildet ist, dass das erste Bedienelement mittels eines Daumens einer den Umgreifungskörper umschließenden Hand betätigbar ist, und
dass der Schubmechanismus wenigstens ein zweites Bedienelement vorsieht, das am Umgreifungskörper derart ergonomisch angeordnet und ausgebildet ist, dass das zweite Bedienelement mittels eines Zeige- oder Mittelfingers einer den Umgreifungskörper umschließenden Hand betätigbar ist.

6. Medizinisches Instrument nach Anspruch 5,
wobei das zweite Bedienelement in Art eines am Umgreifungskörper angebrachten Hebels ausgebildet ist, der über eine im Umgreifungskörper angeordnete Getriebeeinheit mit einer Schub-Zug-Stange kinematisch verbunden ist, die lösbar fest mit dem proximalen Bereich des ETT gefügt ist, und
dass die Getriebeeinheit bei proximalwärtiger Auslenkung des Hebels in Richtung des Umgreifungskörpers die Schub-Zug-Stange sowie den mit dieser lösbar fest gefügten ETT distalwärts und bei entgegen gerichteter Auslenkung des Hebels die Schub-Zug-Stange sowie den mit dieser lösbar fest gefügten ETT proximalwärts schiebt.

7. Medizinisches Instrument nach Anspruch 6,
wobei die Getriebeeinheit ein um eine Drehachse drehbar gelagertes Zahnrad mit Außenverzahnung vorsieht, in die eine mit dem Hebel verbundene Zahnstange sowie die in Art einer Zahnstange ausgebildete Schub-Zug-Stange eingreifen.

8. Medizinisches Instrument nach einem der Ansprüche 2 bis 7,
wobei das Führungselement zumindest bereichsweise aus einem biegeelastischen Material gefertigt ist, über eine Längserstreckung vom proximalen zum distalen Elementende mit einer dem Führungselement zugeordneten Längsachse verfügt sowie am distalen Elementende eine Befestigungsstruktur vorsieht, mit einer symmetrisch zur Längsachse orthogonal orientierten Erstreckung, und
dass die distalen Elementenden der zwei Zugkräfte übertragenden Strangabschnitte an jeweils quer zur Längsachse beabstandeten Befestigungsstellen an der Befestigungsstruktur angelenkt sind.

9. Medizinisches Instrument nach Anspruch 1,
wobei das bidirektional verformbare Führungselement eine Baueinheit aufweist, die durch elektrische, pneumatische oder hydraulische Steuersignale eine Formänderung des Führungselementes initiiert,
dass das Steuermittel einen mit der Baueinheit verbundenen Schaltkreislauf vorsieht, und
dass der an der Bedieneinheit angebrachte Aktuator Teil des Schaltkreislaufes ist.

10. Medizinisches Instrument nach Anspruch 9,
wobei die Baueinheit wenigstens eine der nachfolgenden Komponenten umfasst: mechatronische Baueinheit, elektromotorische Getriebeeinheit, Aktor-Sensor-Einheit, piezoelektrischer Werkstoff, magnetostriktiver Werkstoff, Formgedächtnislegierung, Blähkörper, Fluidleitung.

11. Medizinisches Instrument nach einem der Ansprüche 1 bis 10,
wobei der Führungsstab von einem Lumen durchsetzt ist, durch das das Steuermittel verläuft.

12. Medizinisches Instrument nach Anspruch 11,
wobei längs des Lumens oder eines weiteren Lumens durch den Führungsstab sowie eines sich längs des Führungselementes erstreckenden Lumens ein Beobachtungsmittel geführt ist.

13. Medizinisches Instrument nach Anspruch 12,
wobei das Beobachtungsmittel in Art wenigstens eines der nachfolgenden Komponenten ausgebildet ist: Beleuchtungskatheter, Kamerakatheter, LIDAR-Katheter, Sensorkatheter.

## Claims

1. A medical instrument for the intubation of a patient, with an endotracheal tube, ETT for short, enclosing a hollow channel, together with a guiding rod (1), which is arranged along the hollow channel, and is plastically deformable at least in an axial portion, with a distal (4) and proximal (2) rod end, to the distal rod end of which a bidirectionally deformable guiding element (5) is attached, with a distal and a proximal element end, the proximal element end of which is joined to the distal rod end, and which is operatively connected to a control means,
wherein,
an operating unit (3) designed in the form of a handle is attached to the proximal rod end of the guiding rod, with an actuator (16) connected to the control means, and with a push-pull mechanism, which is securely in operative connection in a releasable manner with the ETT, and by means of which the ETT can be moved along the guiding rod,
the handle has a longitudinal extent that includes an angle α with respect to a longitudinal extension that can be assigned to the ETT, where 90° ≤ α ≤ 135°,
that the push-pull mechanism and the proximal region of the ETT partially overlap on the longitudinal extent of the ETT, and
in the course of intubation of the ETT, together with the guiding rod arranged in the hollow channel of the ETT, with the bidirectionally deformable guiding element attached to it, the distal end of the ETT finishes with the distal end of the element, or the proximal end of the element merely projects beyond the distal end of the ETT in the form of a dome, so that when the guide element is deformed, the ETT can actively deform along its distal end region..

2. The medical instrument in accordance with Claim 1,
wherein the control means has at least two linear elements transmitting tensile forces, each with a linear element end fixed to the distal element end of the bidirectionally deformable guiding element, together with in each case a linear end section located opposite the linear element end fixed to the guiding element, which end section is, or can be, brought into operative connection with the actuator, such that when the actuator is actuated, both linear elements can be kinematically deflected in opposite directions to one another.

3. The medical instrument in accordance with Claim 2,
wherein the actuator has a rotary element mounted such that it can rotate about an axis of rotation, on which the linear end sections of both linear elements are in operative connection with the rotary element in each case in half-planes located opposite the axis of rotation.

4. The medical instrument in accordance with Claim 2 or 3,
wherein the two linear elements transmitting tensile forces are connected to each other in one piece at the linear end sections.

5. The medical instrument in accordance with one of the Claims 1 to 4,
wherein the handle has an ergonomically shaped gripping body,
in that, the actuator provides at least one first operating element, which is ergonomically arranged and designed on the gripping body, such that the first operating element can be actuated by means of a thumb of a hand enclosing the gripping body, and
the pushing mechanism provides at least one second operating element, which is ergonomically arranged and designed on the gripping body, such that the second operating element can be actuated by means of an index or middle finger of a hand enclosing the gripping body.

6. The medical instrument in accordance with Claim 5,
wherein the second operating element is designed in the form of a lever attached to the gripping body, which is kinematically connected via a transmission unit arranged in the gripping body to a push-pull rod, which is releasably fixed to the proximal section of the ETT, and
the transmission unit pushes the push-pull rod, and the ETT detachably connected to it, in the distal direction when the lever is deflected proximally in the direction of the gripping body, and when the lever is deflected in the opposite direction, the push-pull rod and the ETT detachably connected to it are pushed in the proximal direction.

7. The medical instrument in accordance with Claim 6,
wherein the transmission unit provides a gearwheel with external teeth, which is mounted such that it can rotate about an axis of rotation, and with which a toothed rack connected to the lever, and the push-pull rod designed in the form of a toothed rack, engage.

8. The medical instrument in accordance with one of the Claims 2 to 7,
wherein the guiding element is made of a flexurally elastic material at least in some regions, has a longitudinal extent from the proximal to the distal element end with a longitudinal axis assigned to the guiding element, and provides an attachment structure at the distal element end with an extent oriented symmetrically orthogonal to the longitudinal axis, and
the distal element ends of the two tensile force-transmitting linear elements are articulated to the attachment structure at attachment points spaced at right angles to the longitudinal axis.

9. The medical instrument in accordance with Claim 1,
wherein the bidirectionally deformable guiding element has a structural unit that initiates a change in shape of the guiding element by means of electrical, pneumatic, or hydraulic, control signals,
the control means provides a circuit connected to the unit, and
the actuator attached to the operating unit is part of the circuit.

10. The medical instrument in accordance with Claim 9,
wherein the structural unit comprises at least one of the following components: a mechatronic structural unit, an electromotive transmission unit, an actuator-sensor unit, a piezoelectric material, a magnetostrictive material, a shape memory alloy, an inflatable body, a fluid line.

11. The medical instrument in accordance with one of the Claims 1 to 10,
wherein the guiding rod is penetrated by a lumen, through which the control means runs.

12. The medical instrument in accordance with Claim 11,
wherein the guiding rod and a lumen extending along the guiding element are guided by an observation medium along the lumen or a further lumen.

13. The medical instrument in accordance with Claim 12,
wherein the observation medium is designed as at least one of the following components: an illumination catheter, a camera catheter, a LIDAR catheter, a sensor catheter.

## Revendications

1. Instrument médical, destiné à intuber un patient, doté d'un tube endotrachéal, en abrégé TET, englobant un canal creux, ainsi que d'une tige de guidage (1), placée le long du canal creux, plastiquement déformable au moins dans une zone axiale partielle, dotée d'une extrémité de tige distale (4) et proximale (2), sur l'extrémité de tige distale de laquelle est monté un élément de guidage (5) bidirectionnellement déformable, doté d'une extrémité d'élément distale et proximale, dont l'extrémité d'élément proximale est jointée sur l'extrémité de tige distale et qui est en liaison active avec un moyen de commande,
sur l'extrémité de tige proximale de la tige de guidage étant montée une unité de manipulation conçue à la manière d'une poignée, dotée d'un actionneur (16) relié avec le moyen de commande et dotée d'un mécanisme de poussée-traction qui est en liaison active fixe amovible avec le TET, par lequel le TET est déplaçable le long de la tige de guidage,
la poignée détenant une extension longitudinale, qui par rapport à une extension longitudinale susceptible d'être associée au TET, inclut un angle α, avec 90° ≤ α ≤ 135 °,
le mécanisme de poussée-traction et la zone proximale du TET se chevauchant partiellement dans la zone du TET,
lors de l'intubation du TET avec la tige de guidage placée dans le canal creux du TET avec l'élément de guidage bidirectionnellement déformable monté sur celle-ci, l'extrémité distale du TET se terminant sur l'extrémité d'élément distale ou l'extrémité d'élément proximale ne saillant au-delà de l'extrémité distale du TET qu'à la manière d'une coupe, de sorte que lors d'une déformation de l'élément de guidage, le TET soit activement co-déformable le long de sa zone d'extrémité distale.

2. Instrument médical selon la revendication 1,
le moyen de commande comportant au moins deux segments de brin transmettant les forces de traction, dotés chacun d'une extrémité de segment de brin fixée sur l'extrémité d'élément distale de l'élément de guidage bidirectionnellement déformable et chacun d'un segment d'extrémité de brin au vis-à-vis de l'extrémité de segment de brin fixée sur l'élément de guidage, qui est ou est susceptible d'être amené en liaison active avec l'actionneur, de telle sorte que lors d'un actionnement de l'actionneur, les deux segments de brin puissent être cinématiquement déviés, à l'opposée l'un de l'autre.

3. Instrument médical selon la revendication 2,
l'actionneur comportant un élément rotatif, logé en rotation autour d'un axe de rotation, sur lequel, dans des demi-plans placés chacun au vis-à-vis de l'axe de rotation, les segments d'extrémité de brin des deux segments de brins se trouvent en liaison active avec l'élément de rotation.

4. Instrument médical selon la revendication 2 ou 3,
les deux segments de brin transmettant des forces de traction étant reliés en monobloc l'un avec l'autre sur les segments d'extrémité de brin.

5. Instrument médical selon l'une quelconque des revendications 1 à 4,
la poignée comportant un corps d'entourage ergonomiquement façonné,
l'actionneur prévoyant au moins un premier élément de manipulation qui est ergonomiquement placé sur le corps d'entourage et conçu de telle sorte, que le premier élément de manipulation soit actionnable par le pouce d'une main enserrant le corps d'entourage et
le mécanisme de poussée prévoyant au moins un deuxième élément de manipulation qui est ergonomiquement placé sur le corps d'entourage et conçu de telle sorte, que le deuxième élément de manipulation soit actionnable par l'index ou le majeur d'une main enserrant le corps d'entourage.

6. Instrument médical selon la revendication 5,
le deuxième élément de manipulation étant conçu à la manière d'un levier monté sur le corps d'entourage, qui par l'intermédiaire d'une unité de transmission placée dans le corps d'entourage est cinématiquement relié avec une barre de poussée-traction, qui est jointée fixement de manière amovible sur la zone proximale du TET et
lors d'une déviation en direction proximale du levier dans la direction du corps d'entourage, l'unité de transmission poussant la barre de poussée-traction, ainsi que le TET fixement jointé de manière amovible avec celle-ci dans la direction distale et lors d'une déviation du levier en sens opposé, poussant la barre de poussée-traction, ainsi que le TET fixement jointé de manière amovible avec celle-ci dans la direction proximale.

7. Instrument médical selon la revendication 6,
l'unité de transmission prévoyant une roue dentée logée en rotation autour d'un axe de rotation, dotée d'une denture extérieure, dans laquelle s'engagent une crémaillère reliée avec le levier, ainsi que la barre de poussée-traction, conçue à la manière d'une crémaillère.

8. Instrument médical selon l'une quelconque des revendications 2 à 7,
l'élément de guidage étant fabriqué au moins par endroits en une matière élastique en flexion, disposant sur une extension longitudinale de l'extrémité d'élément proximale jusqu'à distale d'un axe longitudinal associé à l'élément de guidage et prévoyant sur l'extrémité d'élément distale une structure de fixation, avec une extension orientée de manière orthogonale par rapport à l'axe longitudinal et
les extrémités d'élément distales des deux segments de brin transmettant des forces de traction étant articulées sur la structure de fixation sur chaque fois deux points de fixation écartés de l'axe longitudinal.

9. Instrument médical selon la revendication 1,
l'élément de guidage bidirectionnellement déformable comportant un module, qui par des signaux de commande électriques, pneumatiques ou hydrauliques, initie un changement de forme de l'élément de guidage,
le moyen de commande prévoyant un circuit de commutation relié avec le module
et
l'actionneur monté sur l'unité de manipulation étant une partie du circuit de commutation.

10. Instrument médial selon la revendication 9,
le module comprenant au moins l'un des composants suivants : un module mécatronique, une unité de transmission électro-motorisée, une unité actionneur-capteur, une matière piézoélectrique, une matière magnéto-restrictive, un alliage à mémoire de forme, un corps gonflant, un conduit de fluide.

11. Instrument médical selon l'une quelconque des revendications 1 à 10,
la tige de guidage étant traversée par un lumen à travers lequel s'étend le moyen de commande.

12. Instrument médical selon la revendication 11,
le long du lumen ou d'un lumen supplémentaire, un moyen d'observation étant guidé à travers la tige de guidage et d'un lumen s'étendant le long de l'élément de guidage.

13. Instrument médical selon la revendication 12,
le moyen d'observation étant conçu à la manière d'au moins l'une des composants suivants : un cathéter lumineux, une caméra-cathéter, un cathéter LiDAR, un cathéter à capteur.
